# EUROPÄISCHE PATENTSCHRIFT

(11) **EP 2 660 952 B1**
(45) Veröffentlichungstag und Bekanntmachung des Hinweises auf die Patenterteilung: **21.03.2018**
(21) Anmeldenummer: 13165381.8
(22) Anmeldetag: 25.04.2013
(51) Int. Cl.: H02J 50/12

(54) **Energieübertragungssystem**
Energy transfer system
Système de transfert d'énergie

(30) Priorität: 04.05.2012 DE 102012103942
(43) Veröffentlichungstag der Anmeldung: 06.11.2013
(73) Patentinhaber: WITTENSTEIN SE, 97999 Igersheim (DE)
(72) Erfinder: Grödl, Felix, 83624 Otterfing (DE); Friedmann, Jan, 86920 Denklingen (DE)
(74) Vertreter: Zimmermann & Partner Patentanwälte mbB

(56) Entgegenhaltungen:
- US-A1- 2004 130 916
- US-A1- 2010 308 939

## Beschreibung

### Gebiet der Erfindung

Die Erfindung betrifft ein Verfahren zur drahtlosen Übertragung von Energie nach dem Anspruch 1 und eine Vorrichtung zur drahtlosen Übertragung von Energie nach dem nebengeordneten Anspruch.

### Stand der Technik

Aus dem Stand der Technik sind Energieübertragungssysteme bekannt, welche drahtlos Energie von einem Primärkreis zu einem Sekundärkreis übertragen können. Dabei wird von Geräten aus dem Stand der Technik die Gegeninduktivität von zwei Wicklungen verwendet, um über ein elektromagnetisches Feld Energie zu übertragen.

Aus der WO 2009/091267 A2 ist ein Energieübertragungssystem bekannt, welches eine einstellbare Resonanzfrequenz für einen Primärkreis und einen Sekundärkreis aufweist, um die Übertragung von Energie zu optimieren. Allerdings wird bei der Auswahl der Resonanzfrequenz oder der Sendefrequenz keine Rücksicht darauf genommen, in welchem Betriebszustand sich die Übertragungsvorrichtung oder eine daran angeschlossene Last befindet.

In der US 2010/0308939 A1 sind Systeme zum drahtlosen Übertragen von Energie mit Primärkreis und Sekundärkreis beschrieben, wobei Einstellmöglichkeiten der Eigenschaften eines Schwingkreises vorgeschlagen werden. Die US 2004/0130916 A1 beschreibt Systeme zur elektromagnetischen Energieübertragung.

### Offenbarung der Erfindung

Aufgabe der Erfindung ist es, verbesserte Vorrichtungen und Verfahren zur Energieübertragung anzugeben. Insbesondere soll eine hochgradig zuverlässige und flexible Energieübertragung ermöglicht werden.

Die Aufgabe wird mit einem Verfahren nach Anspruch 1 und einer Vorrichtung nach Anspruch 8 gelöst. Typische Ausführungsformen der Erfindung betreffen ein Verfahren zur Übertragung von Energie von einem Primärkreis, welcher eine Wicklung und eine einstellbare Kapazität zur Einstellung einer Resonanzfrequenz des Primärkreises aufweist, und einen Sekundärkreis, der eine zweite Wicklung umfasst, welche für eine elektromagnetische Interaktion mit der ersten Wicklung anordnebar ist. Typische Verfahren umfassen ein Ermitteln einer Gegeninduktion der ersten Wicklung zu der zweiten Wicklung, ein Einstellen der Kapazität für die Energieübertragung von dem Primärkreis zu dem Sekundärkreis in Abhängigkeit eines Betriebsparameters und der Gegeninduktion, und ein Einstellen einer Sendefrequenz für die Energieübertragung in Abhängigkeit des Betriebsparameters und der Gegeninduktion. Bei typischen Verfahren werden dabei je nach Betriebsparameter und Gegeninduktion für die Sendefrequenz auch abweichende Werte von der Resonanzfrequenz des Primärkreises oder des Sekundärkreises eingestellt. Bei weiteren typischen Ausführungsformen wird die Kapazität für die Energieübertragung von dem Primärkreis zu dem Sekundärkreis derart eingestellt, dass die Resonanzfrequenz des Primärkreises, insbesondere in Abhängigkeit des Betriebsparameters oder der Gegeninduktion, von der Resonanzfrequenz des Sekundärkreises abweicht. Hintergrund ist, dass mit abweichenden Werten für die Resonanzfrequenz des Primärkreises oder der Sendefrequenz Übertragungsfunktionen für die Energieübertragung in Abhängigkeit der Sendefrequenz geschaffen werden können, welche zwei Maxima mit einem dazwischen liegenden Plateau oder allgemein eine Plateaufunktion aufweisen, oder es können Übertragungsfunktionen geschaffen werden, welche einen breiteren "Peak" aufweisen als bei Abstimmung aller Größen auf exakt dieselbe Frequenz. Auf diese Weise kann auch mit einer Sendefrequenz abweichend von einem lokalen Maximum der Übertragungsfunktion gearbeitet werden, da auch abseits des lokalen Maximums ausreichend hohe Werte der Übertragungsfunktion zur Verfügung stehen, um einen ausreichenden Wirkungsgrad zu erreichen. Unter Umständen kann bei von der Resonanzfrequenz oder vom Maximum der Übertragungsfunktion abweichender Sendefrequenz eine höhere Energieübertragung erfolgen als bei Wahl des Maximums oder der Resonanzfrequenz.

Typischerweise umfasst der Betriebsparameter eine Information über eine Last des Sekundärkreises oder eine Information über eine Energiequelle des Primärkreises. So können beispielsweise bei einer Last, welche über einen bestimmten Lastschwellenwert liegt, oder bei Zuständen der Energiequelle, welche eine Speisung aus einer mobilen Energiequelle bedeuten, die Kapazität oder die Sendefrequenz eingestellt werden, sodass ein Übertragungswirkungsgrad optimiert wird, oder sodass die Menge einer übertragbaren Leistung optimiert wird. Weitere Informationszustände für die Information über die Last können beispielsweise sein, dass die Last unterhalb eines Lastschwellenwertes liegt, sodass eine Kapazität oder eine Sendefrequenz gewählt werden, welche eine robustere Übertragung ermöglichen. Dabei bedeutet der Ausdruck "robuste Übertragung", dass die Kapazität nicht für einen maximalen Wirkungsgrad bei der Übertragung ausgewählt wird, sondern für eine möglichst breite Übertragungsfunktion, also beispielsweise für eine Übertragungsfunktion, welche ein Plateau aufweist oder zumindest zwei lokale Maxima. Der Ausdruck "Übertragungsfunktion" bezeichnet dabei die Relation von Sekundärstrom, das heißt der Strom im Sekundärkreis, zu Primärspannung, das ist die Spannung im Primärkreis, aufgetragen über die Sendefrequenz. Durch Variation der einstellbaren Kapazität können verschiedene Übertragungsfunktionen geschaffen werden. Das Vorgehen bietet den Vorteil, dass für unterschiedliche Betriebssituationen und Betriebsparameter verschiedene Charakteristika der Übertragungsfunktion ausgewählt werden können.

Bei typischen Verfahren erfolgt eine Übertragung von Energie mit der eingestellten Kapazität und der eingestellten Sendefrequenz und einer Kontrolle oder Regelung der übertragenen Leistung durch Einstellen der Übertragungsspannung, insbesondere sodass eine übertragene Leistung zumindest einer benötigten Leistung der Last am Sekundärkreis entspricht. Typischerweise wird die Spannung im Primärkreis eingestellt. Typische Vorrichtungen der Erfindung umfassen eine Kontrolleinheit, welche Teil einer geschlossenen Regelschleife für die übertragene Leistung ist. Hierzu weisen typische Vorrichtungen einen RF- oder Funk-Übertragungskanal auf, welcher Informationen über den Sekundärstrom oder die Last am Sekundärkreis zu der Kontrolleinheit übermittelt. Die Informationen werden typischerweise mit Hilfe von Sensoren erhalten oder aus anderen Informationen ermittelt. Bei weiteren Ausführungsformen kann ein zweiter RF-Übertragungskanal vorgesehen sein, um Redundanz zu schaffen. Weiterhin ist es möglich, bei Ausführungsformen einen Informationsübertragungskanal über die Energieübertragungsstrecke zu bilden. Dies bedeutet typischer Weise, dass die erste Wicklung und die zweite Wicklung und die darüber übertragene Energie mit dem elektromagnetischen Feld zusätzlich genutzt werden, um Informationen zu übertragen.

Es sollte berücksichtigt werden, dass bei typischen Ausführungsformen der Sekundärkreis für eine Verwendung in einer Maschine vorgesehen ist oder in einer implantierbaren Vorrichtung aufgenommen ist, sodass eine Änderung von Parametern oder Einstellungen des Sekundärkreises im Betrieb unter Umständen schwierig ist. Typische Verfahren eignen sich zur transkutanen Energieübertragung ebenso wie typische Vorrichtungen für eine transkutane Energieübertragung (transcutaneous energy transfer) geeignet sind. Weitere typische Ausführungsformen eignen sich für einen Betrieb außerhalb des menschlichen Körpers. Bei Verwendung eines zweiten RF-oder Funk-Übertragungskanals bietet die Auswahl einer zweiten Übertragungsfrequenz, welche verschieden zu der Übertragungsfrequenz des ersten RF- oder Funkkanals ist, den Vorteil einer höheren Redundanz bei elektromagnetischen Störwellen. Eine Übertragung von Information über die Energieübertragungsstrecke kann realisiert werden durch eine kapazitive Lastmodulation im Primärkreis. Weitere Möglichkeiten sind andere Verfahren, welche die Sendefrequenz mit einer hochfrequenten Informations-tragenden Frequenz überlagern.

Bei typischen Ausführungsformen wird die Kapazität oder die Sendefrequenz anhand einer Lookup-Tabelle bestimmt. Die Lookup-Tabelle bietet den Vorteil, dass Rechenzeit eingespart werden kann. Die Lookup-Tabelle enthält typischer Weise Informationen darüber, welche Kapazität und welche Sendefrequenz für eine gegebene Übertragungscharakteristik oder für eine gegebene Gegeninduktivität und üblicherweise auch in Abhängigkeit des Betriebsparameters eingestellt werden. Nach Einstellen der Sendefrequenz und der Kapazität anhand der Lookup-Tabelle in Abhängigkeit der Gegeninduktivität genügt es bei typischen Ausführungsformen, die Sendeleistung durch Einstellen der Primärspannung anhand des geschlossenen Regelkreises zu regeln. Der geschlossene Regelkreis weist als Eingangsgröße üblicher Weise den Primärstrom auf.

Bei typischen Ausführungsformen wird während der Übertragung von Energie von dem Primärkreis zu dem Sekundärkreis ein Wirkungsgrad der Übertragung ermittelt. Dies kann beispielsweise dadurch geschehen, dass über den Sekundärstrom die Leistung an der Last ermittelt wird und mit einer Sendeleistung des Primärkreises verglichen wird, wobei es auch möglich ist, aus der Primärspannung und dem Primärstrom auf den Wirkungsgrad zu schließen. Typische Vorrichtungen weisen eine Überwachungseinrichtung auf, welche während einer Energieübertragung den Wirkungsgrad überwacht. Dies bietet den Vorteil, dass bei einem Abfallen des Wirkungsgrades unter einen Schwellenwert Maßnahmen ergriffen werden können, um den Wirkungsgrad wieder zu verbessern.

Bei üblichen Verfahren wird überprüft, ob der Wirkungsgrad einen ersten Schwellenwert unterschreitet und bei Unterschreiten des ersten Schwellenwertes wird eine Änderung der Sendefrequenz oder eine Änderung der Kapazität vorgenommen. Auf diese Weise kann mit einfachen Mitteln der Wirkungsgrad wieder verbessert werden. Für das Ändern der Sendefrequenz oder das Ändern der Kapazität kann wiederum eine Lookup-Tabelle verwendet werden, um Rechenzeit einzusparen.

Üblicherweise wird überprüft, ob der Wirkungsgrad einen zweiten Schwellenwert unterschreitet, wobei der zweite Schwellenwert niedriger ist als der erste Schwellenwert, und die Sendefrequenz und die Kapazität geändert, falls die Überprüfung ein Unterschreiten des zweiten Schwellenwertes ergibt. Die Änderung beider genannter Größen bietet den Vorteil, dass der Wirkungsgrad über einen großen Bereich wieder verbessert werden kann.

Bei weiteren Verfahren der Erfindung wird überprüft, ob der Wirkungsgrad einen dritten Schwellenwert unterschreitet, wobei der dritte Schwellenwert niedriger ist als der erste Schwellenwert und gegebenenfalls auch niedrigerer ist als der zweite Schwellenwert. Bei Unterschreiten des dritten Schwellenwertes wird typischer Weise eine Änderung der relativen Position der ersten Wicklung zu der zweiten Wicklung vorgenommen oder initiiert, um die Gegeninduktivität oder den Wirkungsgrad zu verbessern. Dabei ist allgemein in dieser Anmeldung von dem Begriff "Ändern der Position" insbesondere auch umfasst, dass ein Benutzer auf eine erforderliche oder bevorzugte Positionsänderung hingewiesen wird. Ob der Benutzer letztendlich die Position tatsächlich verändert, ist hierbei typischer Weise für das Verfahren, welches durch Ausführungsformen ausgeführt wird oder durch Kontrolleinheiten von Ausführungsformen ausführbar ist, nicht relevant. Weiterhin kann jedoch vorgesehen sein, dass der Benutzer bei Nichtdurchführen von vorgeschlagenen Positionsänderungen durch Warnsignale alarmiert wird, beispielsweise durch akustische Warntöne oder durch ein Vibrieren auf drohende Gefahren durch einen weiteren Abfall des Wirkungsgrades hingewiesen wird. Das Ändern der relativen Position der ersten Wicklung zu der zweiten Wicklung bietet dabei den Vorteil, dass auf diese Weise besonders effektiv der Wirkungsgrad verbessert werden kann.

Bei typischen Verfahren wird die relative Position durch eine Triangulation ermittelt. Eine Triangulation erfolgt dabei üblicher Weise durch Bestimmen der Gegeninduktivität an drei verschiedenen Positionen der ersten Wicklung. Üblicher Weise ist die Position der zweiten Wicklung vorgegeben, sodass die erste Wicklung variiert wird. Bei typischen Verfahren wird ein Benutzer aufgefordert, die erste Wicklung gemäß Vorgaben oder gemäß seinem eigenen Gutdünken zu bewegen, wobei auch eine Automatisierung der Bewegung der ersten Wicklung möglich ist. Weitere Ausführungsformen umfassen zwei, drei oder mehr Wicklungen, um eine Triangulation für einen Benutzer zu erleichtern, so muss beispielsweise bei drei oder mehr Wicklungen für eine Triangulation keine Wicklung durch den Benutzer zwingend bewegt werden. Eine einzige Wicklung weist den Vorteil eines geringeren Gewichts und eines einfacheren Aufbaus auf. Zwei Wicklungen können einen Kompromiss darstellen.

Bei typischen Verfahren wird die optimale Position auf einer vorgegebenen Fläche bestimmt, wobei die vorgegebene Fläche mögliche Positionen für die erste Wicklung umfasst. Bei typischen Ausführungsformen ist die vorgegebene Fläche eine ungekrümmte Fläche. Bei üblichen Ausführungsformen kann eine zylinderförmige oder eine ellipsoide Fläche vorgegeben werden. Typischer Weise wird die Triangulation auf Positionen der vorgegebenen Fläche durchgeführt. Bei typischen Ausführungsformen wird anfänglich oder bei einer Initialisierung eine grundlegende Karte für die Gegeninduktivität berechnet. Eine solche Karte ist üblicherweise gültig für einen bestimmten Übertragungsweg, ein solcher bestimmter Übertragungsweg kann beispielsweise eine bestimmte Implantierungstiefe oder ein Abstand der zweiten Wicklung relativ zu der Fläche sein. Bei typischen Verfahren wird die Karte außerhalb eines menschlichen Körpers berechnet, insbesondere für eine zuvor bereits bekannte Implantierungstiefe. Auf diese Weise kann beispielsweise eine Programmierung einer Empfangseinheit mit der zweiten Wicklung erfolgen, bevor diese Empfangseinheit möglicherweise als implantierbare Vorrichtung in einen menschlichen Körper implantiert wird. Durch Berechnung von drei verschiedenen Positionen in der Fläche für die erste Wicklung kann die exakte relative Position bezüglich der zweiten Wicklung durch Triangulation berechnet werden oder durch Nachsehen in einer Karten-Lookup-Tabelle. Eine solche Tabelle bedient den Vorteil, dass Rechenzeit eingespart wird. Auf diese Weise kann die Position für eine ideale geometrische Wicklungsanordnung durch einen Positionsvektor und Abstand bestimmt werden.

Vor dem Einstellen der Kapazität und der Sendefrequenz wird vorzugsweise die erste Wicklung an der optimierten Position positioniert oder es werden Informationen für einen Nutzer zum Positionieren der ersten Wicklung an der optimalen Position ausgegeben. Hierzu kann bei Ausführungsformen von erfindungsgemäßen Vorrichtungen ein Display zur Ausgabe der Informationen vorgesehen sein oder es können optische Pfeile angegeben werden oder es können akustische Signale verwendet werden. Der Nutzer wird damit angewiesen, die erste Wicklung vorzugsweise in der vorgegebenen Fläche zu positionieren.

Bei typischen Vorrichtungen ist der Sekundärkreis in einer implantierbaren Einheit oder Sekundäreinheit angeordnet. Die Sekundäreinheit bezeichnet dabei üblicherweise die Empfangseinheit für die Energieübertragung. Ausgabeeinheiten zur Ausgabe von Positionieranweisungen oder von Informationspositionierung der Wicklungen sind bei typischen Vorrichtungen vorgesehen und können als Display oder optische Ausgabeeinheiten ausgeführt sein.

### Kurze Beschreibung der Zeichnungen

Nachfolgend werden Ausführungsbeispiele anhand der beiliegenden Zeichnungen näher erläutert, es zeigen:
- Fig. 1: eine Skizze einer typischen Vorrichtung der Erfindung;
- Fig. 2 und 3: zeigen Übertragungsfunktionen für verschiedene Betriebszustände einer Last oder einer einstellbaren Kapazität; und
- Fig. 4: zeigt schematisch in einem prinzipiellen Ablaufdiagramm eine Ausführungsform eines Verfahrens.

### Beschreibung von Ausführungsbeispielen

Nachfolgend werden die bevorzugten Ausführungsformen anhand der Figuren beschrieben, wobei die Erfindung nicht auf die Ausführungsbeispiele beschränkt ist, vielmehr wird der Umfang der Erfindung durch die Ansprüche bestimmt. Bei der Beschreibung der Ausführungsform werden unter Umständen in verschiedenen Figuren und für verschiedene Ausführungsformen gleiche Bezugszeichen für gleiche oder ähnliche Teile verwendet, um die Beschreibung übersichtlicher zu gestalten. Dies bedeutet jedoch nicht, dass entsprechende Teile der Erfindung auf die in den Ausführungsformen dargestellten Varianten beschränkt sind.

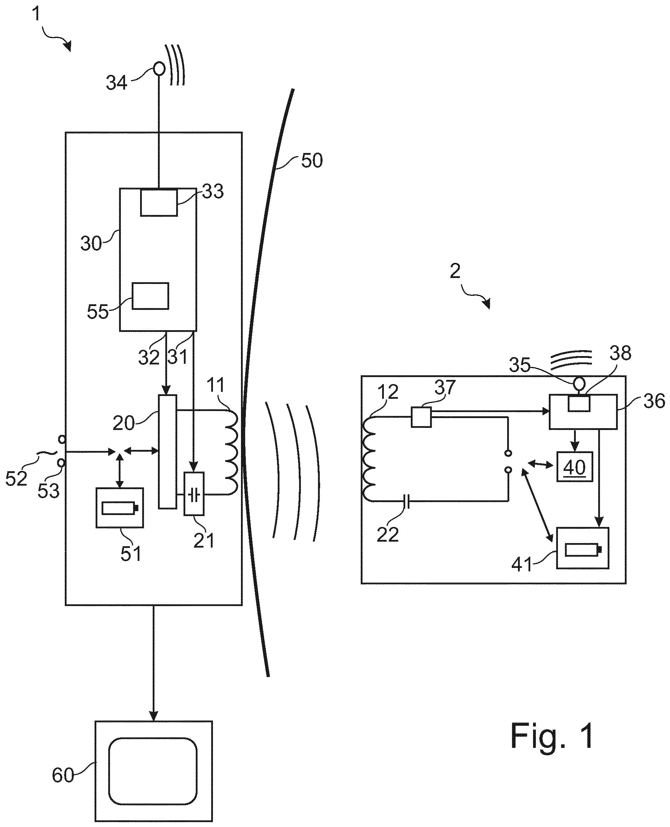

In der Fig. 1 ist eine beispielhafte Ausführungsform gezeigt, welche eine Primäreinrichtung 1 und eine Sekundäreinrichtung 2 umfasst. Die Primäreinrichtung 1 weist einen Primärkreis auf, in welchem eine erste Wicklung 11 angeordnet ist. Die Sekundäreinrichtung 2 weist einen Sekundärkreis auf, in welchem eine zweite Wicklung 12 angeordnet ist.

Bei Ausführungsformen ist die Sekundäreinrichtung dazu vorgesehen, in einer Maschine, beispielsweise an einer schwer zugänglichen Stelle, oder in einem tierischen Körper oder in einem Bereich mit gesundheitsgefährdenden Stoffen angeordnet zu werden. Typische Ausführungsformen der Erfindung sehen insbesondere in der Primäreinrichtung mehr einstellbare, schaltbare oder komplexe Komponenten als in der Sekundäreinrichtung vor, um die Sekundäreinrichtung so einfach wie möglich zu gestalten. Bei weiteren Ausführungsformen sind jedoch auch in der Sekundäreinrichtung schaltbare Komponenten vorgesehen. Sekundäreinrichtungen typischer Ausführungsformen sind implantierbar oder dazu vorgesehen, innerhalb eines menschlichen Körpers oder eines tierischen angeordnet zu werden.

Der Primärkreis weist einen Sendeverstärker 20 mit H-Brückenschaltung auf, welcher typischerweise eine Einstellung der Sendefrequenz ermöglicht. Der Primärkreis weist außerdem im Hauptschluss eine einstellbare Kapazität 21 auf, welche im dargestellten Ausführungsbeispiel als Kondensatormatrix ausgeführt ist. Die Kondensatormatrix weist eine 6-Bit-Schaltung auf, sodass 64 mögliche Kapazitätswerte angesteuert werden können. Bei typischen Ausführungsformen reicht eine 6-Bit-Matrix aus, um eine ausreichende Einstellbarkeit der Resonanzfrequenz des Primärkreises mit der einstellbaren Kapazität 21 und der ersten Wicklung 11 zu gewährleisten. Bei weiteren Ausführungsformen ist eine 7-Bit-Matrix oder mit noch mehr Möglichkeiten ausgestattete Matrix vorgesehen. Weiterhin ist auch möglich, einen stufenlos einstellbaren Kondensator zu verwenden oder eine Kondensatormatrix mit weniger Schaltmöglichkeiten. Der Vollständigkeit halber wird noch auf den zweiten Kondensator 22 des Sekundärkreises hingewiesen, welcher bei typischen Ausführungsformen eine fixe Kapazität aufweist. Auf diese Weise kann die Sekundäreinrichtung 2 möglichst einfach gestaltet werden. Alternativ ist es jedoch auch möglich, auf der Sekundärseite an Stelle des fixen zweiten Kondensators 22 ebenso eine Kondensatormatrix oder einen einstellbaren Kondensator zu verwenden.

Zur Kontrolle, das heißt zum Überwachen, Regeln oder Steuern, verschiedener Funktionen der Primäreinrichtung 1 und gegebenenfalls auch der Sekundäreinrichtung 2 umfasst die Primäreinrichtung 1 eine Kontrolleinheit 30. Die Kontrolleinheit 30 kann über Signalausgänge 31 und 32 die Sendefrequenz des Verstärkers 20 als auch die Kapazität der Kondensatormatrix 21 einstellen. Weiterhin umfasst die Kontrolleinheit 30 eine Radiofrequenz-Übertragungseinheit 33, welche über eine erste Antenne 34 über Radiofrequenzwellen kommunizieren kann. Analog hierzu weist die Sekundäreinrichtung eine zweite Radiofrequenzantenne 35 auf, welche mit einer zweiten Übertragungseinheit 38 verbunden ist, die in einem Kontrollblock 36 angeordnet ist. Der Kontrollblock 36 ist Teil der Sekundäreinrichtung 2 und dient insbesondere dazu, den Strom im Sekundärkreis über einen Stromsensor 37 zu erfassen. Weiterhin ist der Kontrollblock 36 dazu geeignet, eine Verbindung des Sekundärkreises mit einer Last 40 oder eine Verbindung des Sekundärkreises mit einer zweiten Batterie 41 herzustellen.

Typische Ausführungsformen weisen eine Last oder mehrere verschiedene Lastmodule auf. Eine typische Last ist beispielsweise eine Pumpe, ein Aktuator, ein Impulsgeber oder ein Antrieb für einen Stellantrieb, jeweils insbesondere zum Einsatz in gefährlicher Umgebung oder implantierbar ausgestaltet.

Die Sekundäreinrichtung 2 ist bei dem Ausführungsbeispiel der Fig. 1 von der Primäreinrichtung 1 aus betrachtet hinter einer physikalischen Grenzfläche 50, beispielsweise einer Oberfläche eines Körpers, eines Schutzraumes oder einer Maschine angeordnet. Die Primäreinrichtung 1 kann mit der ersten Wicklung 11 auf der vorgegebenen Fläche 50 bewegt werden, um eine Energieübertragung von der ersten Wicklung 11 auf die zweite Wicklung 12 und damit von der Primäreinrichtung 1 zu der Sekundäreinrichtung 2 zu verbessern. In welcher Abfolge solche Bewegungen bei typischen Ausführungsformen vorgesehen sein können, wird im Zusammengang mit typischen Verfahren erläutert.

Die Primäreinrichtung 1 verfügt weiterhin über eine erste Batterie 51, welche wie die zweite Batterie 41 wieder aufladbar ist. Allerdings ist die erste Batterie 51 mit einer wesentlich größeren Kapazität, typischerweise mit einer mindestens 3-fachen oder 10-fachen Kapazität, ausgestattet als die zweite Batterie 41. Bei typischen Ausführungsbeispielen dient die zweite Batterie 41 dazu, lediglich kurze Zeitspannen zu überbrücken, beispielsweise während der einer Energieübertragung problematisch ist. Die erste Batterie 51 hingegen kann auch dazu verwendet werden, um über einen längeren Zeitraum, während dem die Primäreinrichtung 1 nicht mit einem öffentlichen Versorgungsnetz 52 verbunden ist, die Energieversorgung der gesamten Vorrichtung sicherzustellen. Die Primäreinrichtung 1 umfasst Anschlüsse 53, mit welchen sie an das öffentliche Versorgungsnetz 52 angeschlossen werden kann, beispielsweise zum Betrieb der gesamten Primäreinrichtung 1 und damit über die Energieübertragungsstrecke auch der Sekundäreinrichtung 2 oder auch zum Wiederaufladen der ersten Batterie 51. Die erste Batterie oder das öffentliche Netz stellen die Energieversorgung der Primäreinrichtung dar. Weiterhin umfasst die Primäreinrichtung einen in der Kontrolleinheit 30 angeordneten Speicher 55 zum Abspeichern einer Lookup-Tabelle.

Typische Primäreinrichtungen umfassen einen Speicher, welcher üblicherweise Teil der Kontrolleinheit ist, zum Abspeichern eines Zusammenhangs zwischen der Gegeninduktivität der ersten Wicklung und der zweiten Wicklung einerseits und einer Positionierung der Primäreinrichtung oder der ersten Wicklung auf der Grenzfläche andererseits. Weiterhin wird in der Lookup-Tabelle typischerweise eine Sendefrequenz und eine Kapazität abgespeichert, welche für eine gegebene Gegeninduktivität einen maximalen Wirkungsgrad der Übertragung ergeben. In der Tabelle kann für eine ermittelte Gegeninduktivität nachgeschlagen werden um die Position der Spule zu bestimmen. Die Lookup-Tabelle wird typischerweise mit einer vorangegangenen Simulation erzeugt, welche üblicherweise für verschiedene Tiefen der Sekundärwicklung unter der Grenzfläche erfolgt. Eine weitere Möglichkeit ist, die Lookup-Tabelle nach Einbau der Sekundärwicklung oder der Sekundäreinrichtung unter der Grenzfläche zu kalibrieren oder die Werte durch Messung zu ermitteln.

Die Primäreinrichtung 1 ist mit einer Auswerteinheit 60 verbindbar, über welche optische oder akustische Anweisungen zur Positionierung der Primäreinrichtung 1 auf der Grenzfläche 50 an einen Benutzer ausgegeben werden können.

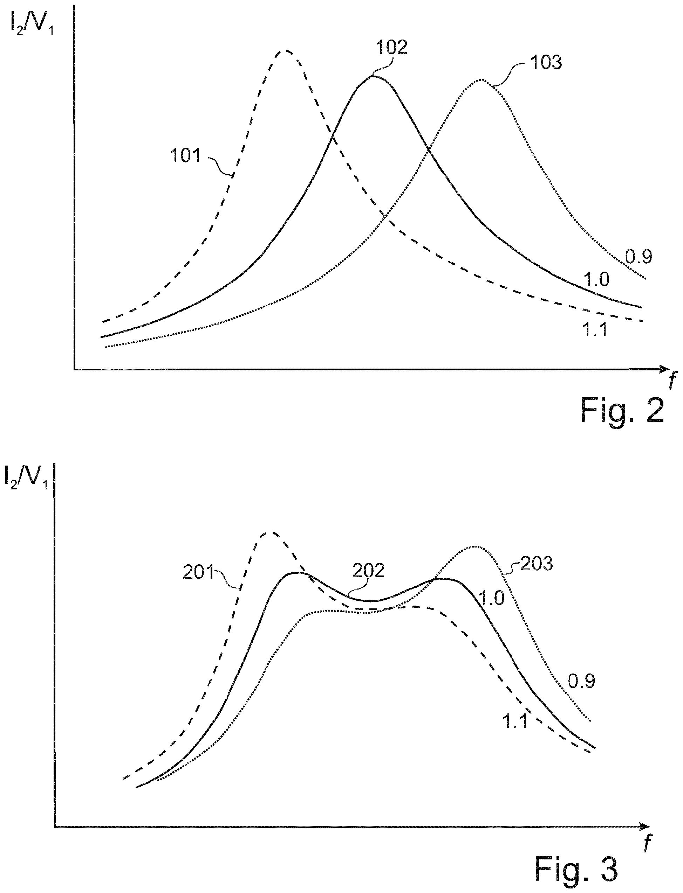

In der Fig. 2 sind verschiedene Übertragungsfunktionen dargestellt. Die Übertragungsfunktionen stellen das Verhältnis des Sekundärstroms I2 in dem Sekundärkreis zu der Primärspannung V1 über die Sendefrequenz f dar. Dabei ist in der Fig. 2 der Fall einer höheren Last im Sekundärkreis dargestellt, wohingegen die Fig. 3 den Fall darstellt, bei welcher die Last lediglich zehn Prozent der Last beträgt, welche in der Fig. 2 dargestellt ist. Die Last kann sich aus unterschiedlichen Gründen vorhersehbar, planbar oder unvorhersehbar ändern. So ist es bei einem Antrieb oder einem Stellmotor möglich, dass sich für unterschiedliche Betriebssituationen, bspw. schnell oder langsam, Beschleunigung oder Abbremsen, unterschiedliche Energieaufnahmen ergeben. Weiterhin kann vorgesehen sein, dass zwischen einem ersten Betriebszustand, bei welchem ein Akku der Sekundäreinrichtung nicht geladen wird, und einem zweiten Betriebszustand, bei welchem der Akku geladen wird, unterschieden wird. Außerdem kann zwischen einem Betriebszustand, bei welchem Netzanschluss der Primäreinrichtung vorliegt und einem Betriebszustand, bei welchem kein Netzanschluss der Primäreinrichtung vorliegt, unterschieden werden. Diese Betriebszustände können durch den Betriebsparameter ausgedrückt werden. Im Ergebnis wird typischerweise eine breitere Übertragungsfunktion gewählt, falls beispielsweise Netzanschluss vorliegt. Bei Netzanschluss kommt es nicht immer auf ein optimales Ausnutzen des besten Wirkungsgrades an.

In den beiden Fig. 2 und 3 sind jeweils drei Übertragungsfunktionen dargestellt, wobei sich die verschiedenen Übertragungsfunktionen in der Wahl der Kapazität in dem Primärkreis unterscheiden. Die Kapazität hat bei dem Ausführungsbeispiel der Fig. 1 das Bezugszeichen 21 und ist als Kondensatormatrix ausgeführt. Dabei bedeutet der Wert 1,0 für die Kapazität nicht, dass die Kapazität derart abgestimmt ist, dass die Resonanzfrequenzen des Sekundärkreises und des Primärkreises aufeinander abgestimmt sind. Vielmehr ist der Wert 1,0 auf eine zunächst willkürlich gewählte Kapazitätsgröße bezogen und die Fig. 2 und 3 sollen veranschaulichen, wie durch unterschiedliche Einstellungen der Kapazität das Übertragungsverhalten vom Primärkreis auf den Sekundärkreis beeinflusst werden kann, insbesondere auch in Abhängigkeit der angeschlossenen Lasten. Die verschiedenen Übertragungsfunktionen für eine höhere Last in der Fig. 2 zeichnen sich durch einen vergleichsweise klaren "Peak" aus. Dabei fällt auf, dass der "Peak", hierin auch als Maximum bezeichnet, bei verschiedenen Konstellationen für die Kapazität nur geringfügige Unterschiede zeigt. Dabei ist auch zu berücksichtigen, dass die strichlierte Übertragungsfunktion 101 für eine um zehn Prozent verringerte Kapazität ein Maximum mit einer deutlich ausgeprägten Spitze aufweist, wohingegen die Maxima der beiden anderen Übertragungsfunktionen 102 (durchgezogene Linie) und 103 (gepunktet) abgeflacht sind. Sollte es also je nach Betriebsmodus und Betriebsparameter nicht auf einen absoluten Höchstwert für das Maximum angekommen, sondern vielmehr ein breiteres Maximum gewünscht sein, beispielsweise bei einem Netzbetrieb, könnte auch die Kapazität so eingestellt werden, dass eine der Übertragungsfunktionen 102 oder 103 gewählt wird. Die Übertragungsfunktionen können für verschiedene Konstellationen der Kapazität und für verschiedene Lasten bei typischen Ausführungsbeispielen in einer Lookup-Tabelle gespeichert sein, um Rechenarbeit zu minimieren.

Bei geringerer Last stellt sich unter Umständen ein sogenanntes doppeltes Maximum ein, zumindest bei geeigneter Auswahl der Kapazität. Die durchgezogene Linie 202 in der Fig. 3 zeigt ein doppeltes Maximum. Ein solches doppeltes Maximum erlaubt es, mit einer größeren Bandbreite für auswählbare Sendefrequenzen zu arbeiten, wobei eine solche Einstellung unter Umständen auch Vorteile bei einem versehentlichen Verrutschen der ersten Wicklung bieten kann. Typische Verfahren weisen das Merkmal auf, dass nach Möglichkeit für eine induktive Energieübertragung von dem Primärkreis auf den Sekundärkreis eine Übertragungsfunktion ausgewählt wird, welche ein doppeltes Maximum oder einen "Double-Peak" aufweist. Hierzu sollte angemerkt werden, dass nicht für jede Last- oder Gegeninduktivitäts-Konfiguration und Positions-Konfiguration eine Kurve mit einem solchen doppelten Maximum möglich ist. Weitere typische Ausführungsformen können eine Übertragung auf einer sogenannten Schulter einer Übertragungsfunktion vorsehen, dies entspricht einem Bereich mit im Wesentlichen horizontaler Tangente neben einem Maximum. Dies ist beispielsweise bei der Übertragungsfunktion mit der strichlierten Linie 201 rechts von dem Maximum dieser Übertragungsfunktion der Fall. Bei der Übertragungsfunktion der punktierten Linie 203 ist dies für Frequenzen links des Maximums der Fall.

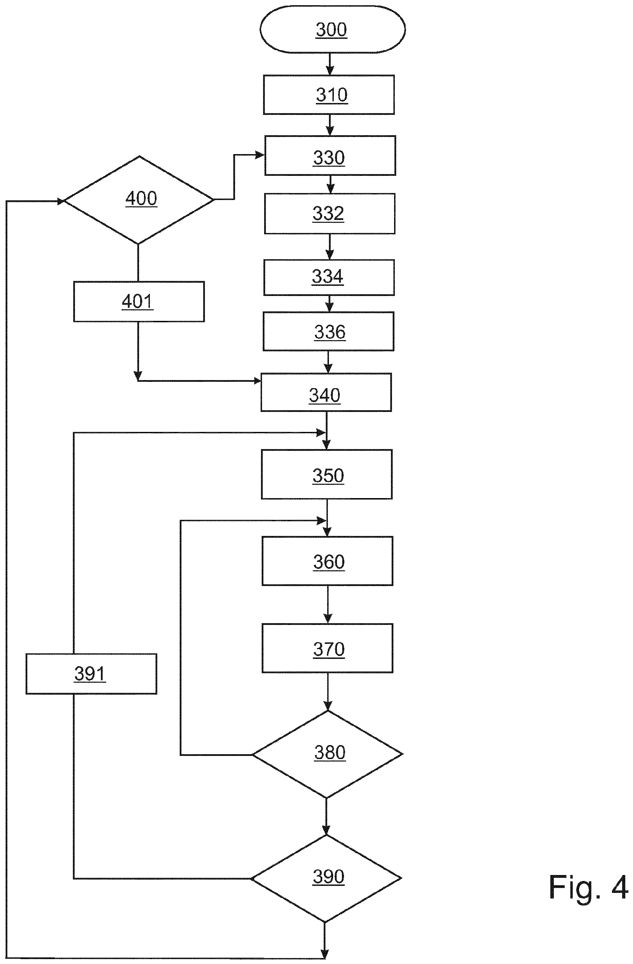

In der Fig. 4 ist ein Ausführungsbeispiel eines Verfahrens gezeigt. Bei der Erläuterung des Verfahrens wird auf die in der Fig. 1 gezeigte Ausführungsform Bezug genommen. Grundsätzlich ist das Verfahren jedoch auch mit anderen Ausführungsformen durchführbar.

Das Verfahren startet mit einem Schritt 300. Nachfolgend wird im Schritt 310 der Strom im Sekundärkreis ermittelt und mit der Spannung im Primärkreis oder mit einer Sendeleistung des Primärkreises verglichen und ein Wirkungsgrad der Energieübertragung berechnet. Der Kontrollblock der Sekundäreinrichtung wird verwendet, um über die Radiofrequenzübertragung den ermittelten Wert für den Strom zu der Kontrolleinheit der Primäreinrichtung zu übermitteln. Die Kontrolleinheit ist typischerweise zur Berechnung eines Wirkungsgrades der Energieübertragung von dem Primärkreis zu dem Sekundärkreis eingerichtet.

Typische Ausführungsformen weisen ein zusätzliches oder vorgeschaltetes Verfahren auf, in welchem eine Karte oder Lookup-Tabelle für die Gegeninduktion der ersten Wicklung mit der zweiten Wicklung in Abhängigkeit der Position der ersten Wicklung auf einer Oberfläche erstellt wird. Diese in der Lookup-Tabelle gespeicherte Karte bezieht sich üblicherweise auf eine Oberfläche oder Grenzfläche, auf welcher die Primäreinrichtung bewegbar ist. Für verschiedene Orte auf dieser Grenzfläche werden Werte für die Gegeninduktion berechnet oder im Versuch ermittelt und in der Lookup-Tabelle in einem Speicher der Kontrolleinheit abgespeichert.

In einem Schritt 332 wird für mindestens drei verschiedene Positionen der ersten Wicklung die Gegeninduktivität zu der zweiten Wicklung ermittelt. Bei typischen Ausführungsformen wird die Gegeninduktivität ermittelt, indem der Sekundärstrom in der zweiten Wicklung gemessen wird und hieraus die Gegeninduktivität berechnet wird. Üblicherweise wird ein Benutzer dazu aufgefordert, mindestens drei vorgegebene oder frei wählbare Positionen anzusteuern. Sollten die Messungen an den mindestens drei Positionen nicht ausreichen, wird der Benutzer typischerweise aufgefordert, weitere, unterschiedliche Positionen mit der Primäreinrichtung anzusteuern. Mehr als drei verschiedene Positionen können eine höhere Genauigkeit herstellen. Eine weitere Möglichkeit ist, mehrere Wicklungen, bspw. drei, in der Primäreinrichtung vorzusehen oder zusätzliche, an die Primäreinrichtung anschließbare Wicklungen vorzusehen, typischerweise eine oder mindestens zwei zusätzliche Wicklungen. Auf diese Weise kann die Messung beschleunigt werden.

In einem Schritt 334 werden die ermittelten Werte mit der Karte für die Gegeninduktion verglichen oder es wird die Position an Hand der Lookup-Tabelle ermittelt. Die Gegeninduktion hängt neben dem radialen Versatz, bspw. an einer Grenzfläche, auch vom Abstand der beiden Spulen ab. Wird der Abstand der Spulen, auch als Tiefe unter der Grenzfläche bezeichnet, nicht bestimmt, ist die Position der Spule auf Basis der Messung der Gegeninduktivität nicht eindeutig. Somit ergeben sich mehrere, geschichtete Karten, die für den jeweiligen Spulenabstand ausgewählt werden müssen. Wird die Spule an den Ort des absoluten Gegeninduktivitätsmaximums bewegt, kann auf die Tiefe geschlossen werden, und die geeignete Karte gewählt werden. Die Karte ist für die Grenzfläche oder Oberfläche typisch, auf welcher die Primäreinrichtung oder die erste Wicklung bewegt wurde. Bei einigen Ausführungsformen ist die erste Wicklung in der Primäreinrichtung integriert, bei anderen Ausführungsformen ist die erste Wicklung lediglich mit der Primäreinrichtung über eine elektrische Verbindung gekoppelt und kann unabhängig von dieser bewegt werden.

Mit Hilfe der ermittelten Karte wird eine optimale Position für die erste Wicklung bestimmt und die erste Wicklung positioniert (336) und bei typischen Ausführungsformen dort fixiert. Typischerweise wird ein Benutzer über ein Ausgabegerät, das bspw. optisch oder akustisch sein kann, dazu angeleitet, die erste Wicklung oder die Primäreinrichtung zu positionieren, so dass ein Maximum für die Gegeninduktivität zumindest im Wesentlichen, bspw. 80%, 90% oder 95% des Maximums, erreicht werden. Mit der Positionierung der ersten Wicklung ist das Unterverfahren abgeschlossen.

Nach Abschluss der Schritte 330-336 wird die Kapazität, welche in dem Ausführungsbeispiel als Kondensatormatrix verwirklicht ist, in Abhängigkeit eines Betriebsparameters eingestellt, 340. Der Betriebsparameter ist typischerweise die Art oder Größe einer Last oder eine Art der Stromversorgung der Primäreinrichtung, beispielsweise ob eine Versorgung über das öffentliche Netz oder über die wiederaufladbare Batterie vorliegt. Weitere typische Entscheidungskriterien werden an anderer Stelle dieser Anmeldung erläutert. Dies gilt analog für die nachfolgende Auswahl der Sendefrequenz in Schritt 350, wobei die Sendefrequenz typischerweise derart gewählt wird, dass sie nicht notwendigerweise mit der Resonanzfrequenz des Primärkreises übereinstimmt.

Anschließend tritt das Verfahren in einen geschlossenen Regelkreis ein, in welchem die Sendeleistung des Primärkreises durch Ansteuern des Verstärkers in Abhängigkeit des ermittelten Stroms im Sekundärkreis angepasst wird, 360. Weiterhin wird aus dem ermittelten Strom im Sekundärkreis und der Sendeleistung unter Berücksichtigung der Größe der Last ein Wirkungsgrad berechnet, 370. Liegt der Wirkungsgrad über einem ersten Schwellenwert von 80%, bleibt das Verfahren im Regelkreis, 380.

Unterschreitet der Wirkungsgrad den ersten Schwellenwert, springt das Verfahren zu dem Schritt 390, wo überprüft wird, ob der Wirkungsgrad auch einen zweiten Schwellenwert in Höhe von 65% unterschreitet. Wird der zweite Schwellenwert nicht unterschritten, d. h. im Beispiel liegt der Wirkungsgrad bei mindestens 65% aber unter 80%, springt das Verfahren zu dem Schritt 391 und nachfolgen zu Schritt 350. In Schritt 391 wird die Gegeninduktivität bestimmt, um im Schritt 350 die Sendefrequenz anzupassen, so dass der Wirkungsgrad verbessert wird.

Unterschreitet der Wirkungsgrad auch den zweiten Schwellenwert, springt das Verfahren zu dem Schritt 400. Der errechnete Wirkungsgrad wird in dem Schritt 400 mit einem untersten, dritten Schwellenwert von üblicherweise 50% verglichen. Typische dritte Schwellenwerte liegen zwischen 30% und 70%, typischerweise zwischen 35% und 60%. Bei Unterschreiten des dritten Schwellenwertes springt das Verfahren zu dem Schritt 330. Bei einem Wirkungsgrad von mindestens dem dritten Schwellenwert und unterhalb des zweiten Schwellenwertes gelangt das Verfahren über den Schritt 401 zu dem Schritt 340. Im Schritt 401 wird die Gegeninduktivität bestimmt, um im Schritt 340 die Kapazität anzupassen, so dass der Wirkungsgrad verbessert wird.

Typische Werte für den zweiten Schwellenwert liegen zwischen 55% und 80%, typischerweise zwischen 60% und 70%. Übliche Werte für den dritten Schwellenwert liegen zwischen 30% und 70%, typischerweise zwischen 40% und 60%. Grundsätzlich ist der erste Schwellenwert größer als der zweite Schwellenwert und dieser größer als der dritte Schwellenwert.

## Patentansprüche

1. Verfahren zur drahtlosen Übertragung von Energie mit
- einem Primärkreis, der eine erste Wicklung (11) und eine einstellbare Kapazität (21) zur Einstellung einer Resonanzfrequenz des Primärkreises aufweist, und
- einem in einer implantierbaren Vorrichtung aufgenommenen Sekundärkreis, der eine zweite Wicklung (12) umfasst, welche für eine elektromagnetische Interaktion zur Energieübertragung mit der ersten Wicklung anordnebar ist,
mit den Schritten:
- Ermitteln einer Gegeninduktion der ersten Wicklung (11) zu der zweiten Wicklung (12),
- Einstellen der Kapazität (21) in Abhängigkeit eines Betriebsparameters und der ermittelten Gegeninduktion,
- Einstellen einer Sendefrequenz in Abhängigkeit des Betriebsparameters und der Gegeninduktion,
- Ermitteln, während einer Übertragung von Energie von dem Primärkreis zu dem Sekundärkreis, eines Wirkungsgrads der Übertragung,
- Überprüfen, ob der Wirkungsgrad einen ersten Schwellenwert unterschreitet,
- Ändern der Sendefrequenz und/oder Ändern der Kapazität (21), falls die Überprüfung ein Unterschreiten des ersten Schwellenwertes ergibt,
- Überprüfen, ob der Wirkungsgrad einen zweiten Schwellenwert unterschreitet, wobei der zweite Schwellenwert niedriger ist als der erste Schwellenwert,
- Ändern der Sendefrequenz und Ändern der Kapazität (21), falls die Überprüfung ein Unterschreiten des zweiten Schwellenwertes ergibt,
- Überprüfen, ob der Wirkungsgrad einen dritten Schwellenwert unterschreitet, wobei der dritte Schwellenwert niedriger ist als der erste Schwellenwert, und
- Ändern der relativen Position der ersten Wicklung (11) zu der zweiten Wicklung (12), falls die Überprüfung ein Unterschreiten des dritten Schwellenwertes ergibt.

2. Verfahren nach Anspruch 1, **dadurch gekennzeichnet, dass** der Betriebsparameter eine Information über eine Last (40) des Sekundärkreises und/oder eine Information über eine Energiequelle des Primärkreises umfasst.

3. Verfahren nach Anspruch 1 oder 2, **gekennzeichnet durch**
- Übertragen von Energie mit der eingestellten Kapazität und der eingestellten Sendefrequenz und
- Regeln der übertragenen Leistung durch Einstellen der Sendeleistung.

4. Verfahren nach einem der vorhergehenden Ansprüche, **dadurch gekennzeichnet, dass** der Wert für die Kapazität (21) und/oder die Sendefrequenz mittels einer Lookup-Tabelle in Abhängigkeit des Betriebsparameters bestimmt werden.

5. Verfahren nach einem der vorhergehenden Ansprüche, **dadurch gekennzeichnet, dass** eine relative Position der ersten Wicklung (11) zu der zweiten Wicklung (12) durch eine Triangulation ermittelt wird.

6. Verfahren nach einem der vorhergehenden Ansprüche, **gekennzeichnet durch** Bestimmen einer optimalen Position der ersten Wicklung (11) auf einer vorgegebenen Fläche relativ zu der zweiten Wicklung (12) für eine maximierte Gegeninduktion.

7. Verfahren nach Anspruch 6, **dadurch gekennzeichnet, dass** vor dem Einstellen der Kapazität (21) und der Sendefrequenz die erste Wicklung (11) an der optimalen Position positioniert wird und/oder Informationen für einen Nutzer zum Positionieren der ersten Wicklung (11) an der optimalen Position ausgegeben werden.

8. Vorrichtung zur drahtlosen Energieübertragung mit
- einem Primärkreis, der eine erste Wicklung (11) und eine einstellbare Kapazität (21) zur Einstellung einer Resonanzfrequenz des Primärkreises aufweist,
- einem Sekundärkreis, der eine zweite Wicklung (12) umfasst, welche für eine elektromagnetische Interaktion mit der ersten Wicklung (11) anordnebar ist, und
- einer Kontrolleinheit (30), welche dazu eingerichtet ist, ein Verfahren nach einem der vorgehenden Ansprüche durchzuführen.

9. Vorrichtung nach Anspruch 8, **gekennzeichnet durch** eine Ausgabeeinheit (60) zur Ausgabe von Positionieranweisungen an einen Benutzer zur Positionierung der ersten Wicklung (11).

10. Vorrichtung nach Anspruch 8 oder 9, **gekennzeichnet durch** einen Speicher (55) zum Abspeichern eines Zusammenhangs zwischen der Gegeninduktivität der ersten Wicklung (11) und der zweiten Wicklung (12) einerseits und einer Positionierung der Primäreinrichtung oder der ersten Wicklung (11) auf der Grenzfläche (50) andererseits.

## Claims

1. Method for wireless transfer of energy by means of
- a primary circuit comprising a first winding (11) and an adjustable capacitance (21) for adjusting a resonance frequency of the primary circuit, and
- a secondary circuit received in an implantable device and comprising a second winding (12), which can be arranged for an electromagnetic interaction with the first winding for energy transfer,
comprising the steps of:
- determining an inductive coupling of the first winding (11) to the second winding (12),
- adjusting the capacitance (21) based on an operating parameter und the determined inductive coupling,
- adjusting a transmitting frequency based on the operating parameter and the inductive coupling,
- determining, during a transfer of energy form the primary circuit to the secondary circuit, an efficiency of the transfer,
- checking whether the efficiency falls below a first threshold value,
- changing the transmitting frequency and/or changing the capacitance (21) if checking result is below the first threshold value,
- checking whether the efficiency falls below a second threshold value, wherein the second threshold value is lower than the first threshold value,
- changing the transmitting frequency and/or changing the capacitance (21) if checking result is below the second threshold value,
- checking whether the efficiency falls below a third threshold value, wherein the third threshold value is lower than the first threshold value,
- changing the relative position of the first winding (11) to the second winding (12) if checking result is below the third threshold value.

2. Method according to claim 1, **characterized in that** the operating parameter contains information about a load (40) of the secondary circuit and/or information about an energy source of the primary circuit.

3. Method according to claim 1 or 2, **characterized by**
- transferring energy with the adjusted capacitance and the adjusted transmitting frequency,
- regulating the transferred power by adjusting the transmitting power.

4. Method according to anyone of the preceding claims, **characterized in that** the value for the capacitance (21) and/or the transmitting frequency are determined by means of a lookup table based on the operating parameter.

5. Method according to anyone of the preceding claims, **characterized in that** a relative position of the first winding (11) to the second winding (21) is determined by triangulation.

6. Method according to anyone of the preceding claims, **characterized by** determining an optimum position of the first winding (11) on a predefined surface relative to the second winding (12) for maximized inductive coupling.

7. Method according to claim 6, **characterized in that**, prior to adjusting the capacitance (21) and the transmitting frequency, the first winding (11) is positioned at the optimum position and/or information is output to a user for positioning the first winding (11) at the optimum position.

8. Device for wireless energy transfer by means of
- a primary circuit comprising a first winding (11) and an adjustable capacitance (21) for adjusting a resonance frequency of the primary circuit, and
- a secondary circuit comprising a second winding (12) which can be arranged for an electromagnetic interaction with the first winding, and
- a control unit (30) configured to execute a method according to anyone of the preceding claims.

9. Device according to claim 8, **characterized by** an output unit (60) for outputting positioning instructions to a user for positioning the first winding (11).

10. Device according to claim 8 or 9, **characterized by** a memory (55) for storing a correlation between the inductive coupling of the first winding (11) and the second winding (12), on the one hand, and a positioning of the primary means or the first winding (11) on the boundary surface (50), on the other.

## Revendications

1. Procédé de transfert d'énergie sans fil comprenant
- un circuit primaire qui présente un premier enroulement (11) et une capacité (21) réglable pour le réglage d'une fréquence de résonance du circuit primaire, et
- un circuit secondaire logé dans un dispositif implantable et comprenant un deuxième enroulement (12), lequel est apte à être disposé pour une interaction électromagnétique avec le premier enroulement en vue du transfert d'énergie,
comprenant les étapes de :
- détermination d'une contre-induction du premier enroulement (11) par rapport au deuxième enroulement (12),
- réglage de la capacité (21) en fonction d'un paramètre de fonctionnement et de la contre-induction déterminée,
- réglage d'une fréquence d'émission en fonction du paramètre de fonctionnement et de la contre-induction,
- détermination, pendant un transfert d'énergie du circuit primaire au circuit secondaire, d'un rendement du transfert,
- vérification si le rendement franchit à la baisse une première valeur seuil,
- modification de la fréquence d'émission et/ou modification de la capacité (21) dans le cas où la vérification donne un franchissement à la baisse de la première valeur seuil,
- vérification si le rendement franchit à la baisse une deuxième valeur seuil, sachant que la deuxième valeur seuil est plus basse que la première valeur seuil,
- modification de la fréquence d'émission et modification de la capacité (21) dans le cas où la vérification donne un franchissement à la baisse de la deuxième valeur seuil,
- vérification si le rendement franchit à la baisse une troisième valeur seuil, sachant que la troisième valeur seuil est plus basse que la première valeur seuil, et
- modification de la position relative du premier enroulement (11) par rapport au deuxième enroulement (12) dans le cas où la vérification donne un franchissement à la baisse de la troisième valeur seuil.

2. Procédé selon la revendication 1, **caractérisé en ce que** le paramètre de fonctionnement comprend une information sur une charge (40) du circuit secondaire et/ou une information sur une source d'énergie du circuit primaire.

3. Procédé selon la revendication 1 ou 2, **caractérisé par**
- le transfert d'énergie avec la capacité réglée et la fréquence d'émission réglée et
- le réglage de la puissance transférée par réglage de la puissance d'émission.

4. Procédé selon l'une des revendications précédentes, **caractérisé en ce que** la valeur pour la capacité (21) et/ou la fréquence d'émission sont déterminées au moyen d'un tableau de correspondance en fonction du paramètre de fonctionnement.

5. Procédé selon l'une des revendications précédentes, **caractérisé en ce qu'**une position relative du premier enroulement (11) par rapport au deuxième enroulement (12) est déterminée par une triangulation.

6. Procédé selon l'une des revendications précédentes, **caractérisé par** la détermination d'une position optimale du premier enroulement (11) sur une surface prédéfinie relativement au deuxième enroulement (12) pour une contre-induction maximisée.

7. Procédé selon la revendication 6, **caractérisé en ce que**, avant le réglage de la capacité (21) et de la fréquence d'émission, le premier enroulement (11) est positionné dans la position optimale et/ou des informations sont sorties pour un usager pour le positionnement du premier enroulement (11) dans la position optimale.

8. Dispositif de transfert d'énergie sans fil comprenant
- un circuit primaire qui présente un premier enroulement (11) et une capacité réglable (21) pour le réglage d'une fréquence de résonance du circuit primaire,
- un circuit secondaire comprenant un deuxième enroulement (12), lequel est apte à être disposé pour une interaction électromagnétique avec le premier enroulement (11), et
- une unité de contrôle (30), laquelle est configurée pour exécuter un procédé selon l'une des revendications précédentes.

9. Dispositif selon la revendication 8, **caractérisé par** une unité de sortie (60) pour la sortie d'instructions de positionnement à un utilisateur pour le positionnement du premier enroulement (11).

10. Dispositif selon la revendication 8 ou 9, **caractérisé par** une mémoire (55) pour la mémorisation d'un rapport entre la contre-inductivité du premier enroulement (11) et du deuxième enroulement (12) d'une part et un positionnement du dispositif primaire ou du premier enroulement (11) sur la surface limite (50) d'autre part.
